(19)

Europäisches
Patentamt
European
Patent Office
Office européen
des brevets

(11) **EP 4 378 387 A1**

(12) **EUROPEAN PATENT APPLICATION**

(43) Date of publication:
**05.06.2024   Bulletin 2024/23**

(21) Application number: **22210706.2**

(22) Date of filing: **30.11.2022**

(51) International Patent Classification (IPC):
***A61B 5/1455*** *(2006.01)*

(52) Cooperative Patent Classification (CPC):
**A61B 5/14552; A61B 5/684**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
 • **TEN KATE, Warner Rudolph Theophile**
   **5656AG Eindhoven (NL)**
 • **BRANDSMA, Ewout**
   **5656AG Eindhoven (NL)**

 • **GELISSEN, Jozef Hubertus**
   **5656AG Eindhoven (NL)**
 • **HUIJGEN, Hendrik**
   **5656AG Eindhoven (NL)**
 • **SCHAEFFERS, Pien**
   **5656AG Eindhoven (NL)**
 • **DE JAGER, Marinus Karel Johannes**
   **5656AG Eindhoven (NL)**
 • **EJUPI, Andreas**
   **5656AG Eindhoven (NL)**

(74) Representative: **Philips Intellectual Property & Standards**
**High Tech Campus 52**
**5656 AG Eindhoven (NL)**

(54) **METHOD, DEVICE, AND SYSTEM TO PROVIDE GUIDANCE FOR MORE OPTIMAL PPG SENSOR PLACEMENT**

(57)     The present disclosure is directed to systems and methods for providing optimal photoplethysmography (PPG) sensor placement instructions to a subject. As described, the systems and methods of the present disclosure minimizes the risk of low signal quality by ensuring proper device placement, supports patient autonomy by providing guided assistance, and reduces the number of wasted sensors due to improper placement.

FIG. 1

EP 4 378 387 A1

Description

FIELD OF THE DISCLOSURE

The present disclosure relates generally to photoplethysmography (PPG) sensors, and more specifically to systems and methods for providing optimal PPG sensor placement instructions to a subject.

BACKGROUND

Vital signs of a person, for example the heart rate (HR), the respiration rate (RR) or the blood oxygen saturation (SpO2), serve as indicators of the current state of a person and as powerful predictors of serious medical events. For this reason, vital signs are extensively monitored in inpatient and outpatient care settings, at home or in further health, leisure and fitness settings. One way of measuring vital signs is plethysmography. Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heartbeat affects transmission and reflectance correspondingly. Accordingly, PPG sensors may be used to monitor several vital signs of subjects, including blood oxygenation (SpO2), pulse rate (PR), respiration rate (RR), and blood pressure (BP), for example, by transmitting light through a portion of the body to measure one or more of these vital signs. However, in order to obtain accurate vital sign measurements, it is important to capture a high-quality signal with a large signal-to-noise ratio (SNR).

SUMMARY OF THE DISCLOSURE

According to an embodiment of the present disclosure, a method for providing optimal photoplethysmography (PPG) sensor placement instructions to a user is described. The method comprises: providing a PPG placement system comprising a plurality of light sources and at least one light sensor, wherein each of the plurality of light sources is spaced relative to the other plurality of light sources; performing one or more iterations of: (i) emitting light from each of the plurality of light sources, after the PPG placement system is placed on a first location on a surface of the subject's body; (ii) detecting, by the at least one light sensor, the emitted light; (iii) determining, by a processor of the PPG placement system, which one of the plurality of light sources is most optimally placed on the surface of the subject's body; and (iv) providing feedback to the user, via a user interface of the PPG placement system, to move the PPG placement system in the direction of the one of the plurality of light sources that is determined to be most optimally placed on the surface of the subject's body.

In an aspect, the method further comprises determining that the PPG sensor is optimally placed on an optimal location on the surface of the subject's body; and providing feedback to the user, via the user interface of the PPG placement system, that the PPG sensor is optimally placed on the optimal location on the surface of the subject's body.

In an aspect, at least one of the plurality of light sources and the at least one light sensor forms a PPG sensor.

In an aspect, the method further comprises obtaining sensor data from the subject via a PPG sensor of the PPG placement system.

In an aspect, the method further comprises the step of determining that the PPG placement system has been placed on a surface of the subject's body.

In an aspect, determining which of the plurality of light sources is most optimally placed on the surface of the subject's body comprises a comparison of signal quality of the emitted light received from each of the plurality of light sources.

In an aspect, the feedback is one or more of audible feedback, haptic feedback, visual feedback, and text-based feedback.

In an aspect, the PPG placement system comprises a plurality of light sensors, the plurality of light sources and plurality of light sensors forming pairs.

In an aspect, light is emitted sequentially from each of the plurality of light sources during the emitting step.

In an aspect, light is emitted from the plurality of light sources by an arrangement of a single light source.

According to another embodiment of the present disclosure, a method for providing optimal photoplethysmography (PPG) sensor placement instructions to a user is described. The method comprises: providing a PPG placement system comprising at least one light source and at least one light sensor, wherein at least one of the at least one light sources and at least one of the at least one light sensor is a PPG sensor; performing, at each of a plurality of different locations on a surface of the subject's body the steps of: (i) emitting light from each of the plurality of light sources; and (ii) detecting, by the at least one light sensor, the emitted light; determining, by a processor of the PPG placement system, which one of plurality of different locations on the surface of the subject's body is a most optimal placement of the PPG placement system for signal quality; providing feedback to the user, via a user interface of the PPG placement system, regarding the most optimal placement of the PPG placement system.

In an aspect, the method further comprises determining that the user has moved the PPG placement system to the most optimal placement.

In an aspect, at least one of the one or more light sources and the at least one light sensor is a PPG sensor.

In an aspect, the method further comprises obtaining sensor data from the subject via a PPG sensor of the PPG placement system,

In an aspect, the feedback is one or more of audible feedback, haptic feedback, visual feedback, and text-based feedback.

In an aspect, the PPG placement system comprises a plurality of light sensors, the plurality of light sources and plurality of light sensors forming pairs.

According to another embodiment of the present disclosure, a photoplethysmography (PPG) placement system is provided. The PPG placement system comprises: a plurality of light sources, wherein each of the plurality of light sources is spaced relative to the other plurality of light sources; at least one light source; a user interface; a processor configured to: (i) direct the plurality of light sources to emit light after the PPG placement system is placed on a first location on a surface of the subject's body; (ii) receive sensor data from the at least one light sensor regarding the emitted light; (iii) determine which one of the plurality of light sources is most optimally placed on the surface of the subject's body; and (iv) direct the user interface to provide feedback to move the PPG placement system in the direction of the one of the plurality of light sources that is determined to be most optimally placed on the surface of the subject's body.

In an aspect, the processor is further configured to determine that the PPG placement system is optimally placed on the optimal location on the surface of the subject's body.

In an aspect, the processor is further configured to receive sensor data from the subject via the PPG sensor of the PPG placement system, including optionally once the processor has determined that the PPG placement system is optimally placed on the optimal location on the surface of the subject's body.

In an aspect, the feedback is one or more of audible feedback, haptic feedback, visual feedback, and text-based feedback.

In an aspect, the PPG placement system comprises a plurality of light sensors, the plurality of light sources and plurality of light sensors forming pairs.

In an aspect, the processor directs the plurality of light sources to emit light sequentially. In an aspect, the processor is configured to determine which of the plurality of light sources is most optimally placed on the surface of the subject's body by comparing signal quality of the emitted light received from each of the plurality of light sources.

According to another embodiment of the present disclosure, a method for providing optimal photoplethysmography (PPG) sensor placement instructions to a user is described. The method comprises: providing a PPG placement system comprising at least one light source and a plurality of light sensors, wherein each of the plurality of light sensors is spaced relative to the other plurality of light sensors; and performing one or more iterations of: (i) emitting light from at least one light source, after the PPG placement system is placed on a first location on a surface of the subject's body; (ii) detecting, by one or more of the plurality of light sensors, the emitted light; (iii) determining, by a processor of the PPG placement system based on the detected emitted light, an optimal placement for a PPG sensor on the surface of the subject's body; and (iv) providing feedback to the user, via a user interface of the PPG placement system, to move the PPG placement system in the direction of the determined optimal placement on the surface of the subject's body.

In an aspect, the method further comprises determining that the PPG sensor is optimally placed on an optimal location on the surface of the subject's body; and providing feedback to the user, via the user interface of the PPG placement system, that the PPG sensor is optimally placed on an optimal location on the surface of the subject's body.

In an aspect, the method further comprises obtaining sensor data from the subject via a PPG sensor of the PPG placement system.

In an aspect, determining an optimal placement for a PPG sensor on the surface of the subject's body comprises a comparison of the emitted light received by each of the plurality of light sensors.

These and other aspects of the various embodiments will be apparent from and elucidated with reference to the embodiments described hereinafter.

BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.

FIG. 1 is a flowchart of a method for providing optimal PPG sensor placement instructions to a user illustrated according to aspects of the present disclosure.

FIG. 2 is a block diagram of a PPG placement system illustrated according to aspects of the present disclosure.

FIG. 3 is a diagram of a PPG placement system illustrated according to aspects of the present disclosure.

FIG. 4 is a block diagram of a controller of a PPG placement system illustrated according to aspects of the present disclosure.

FIG. 5 is a diagram of a subject and a portion of the subject's circulatory system illustrated according to aspects of the present disclosure.

FIG. 6 is a first enlarged view of a portion of FIG. 5 illustrating a PPG placement system in a first position according to aspects of the present disclosure.

FIG. 7 is a second enlarged view of a portion of FIG. 5 illustrating a PPG placement system in a second position according to aspects of the present disclosure.

FIG. 8 is a third enlarged view of a portion of FIG. 5 illustrating a PPG placement system in a third position according to aspects of the present disclosure.

FIG. 9 is a flowchart of another method for providing optimal PPG sensor placement instructions to a user illustrated according to further aspects of the present disclosure.

FIG. 10 is a block diagram of a PPG placement system illustrated according to aspects of the present disclosure.

FIG. 11 is a flowchart of a method for providing optimal PPG sensor placement instructions to a user illustrated according to aspects of the present disclosure.

DETAILED DESCRIPTION OF EMBODIMENTS

As described herein, PPG refers to an optical measurement technique that evaluates a time-variant change of light reflectance or transmission of an area or volume of interest. In particular, the intensity of the reflected (transmitted) light depends on the absorption and scattering properties of the tissues along the light path. For example, the volume of the arteries changes periodically as a result of the pulsations in the blood flow that in turn are caused by the heart beating, which thereby causes periodic changes of the absorption (scattering) of the light path. Accordingly, it is appreciated by the present disclosure that the light intensity sensed by the PPG sensor fluctuates according to the pulsation. The details of this pulsing signal, possibly together with information from other sensors (such as ECG), enables the estimation of abovementioned physiological parameters.

However, the quality of the signal depends on the specific path that the light traverses. Therefore, the quality of the signal obtained by a PPG sensor depends on the location where the sensor has been attached. For example, the light path can pass through an artery, but it may also partially or only slightly pass through one. Furthermore, the light path may pass through smaller arterioles instead of through a larger artery feeding those arterioles. As a result, reduced signal quality can lead to diminished accuracy of the estimation of the vital-sign values.

While a healthcare specialist (e.g., nurse, physician, etc.) with knowledge of a subject's anatomy may be able to place the PPG sensor at an ideal location to obtain a high-quality signal, a layperson (e.g., a patient or subject, etc.) instructed to attach a PPG sensor at home or on their own may not be able to identify a desirable location to position the PPG sensor. Furthermore, even anatomical variations may complicate identifying the ideal location based only on external landmarks.

Accordingly, the present disclosure is directed to systems and methods for providing optimal PPG sensor placement instructions to a subject. As described, the systems and methods of the present disclosure minimizes the risk of low signal quality by ensuring proper device placement, supports patient autonomy by providing guided assistance, and reduces the number of wasted sensors due to improper placement.

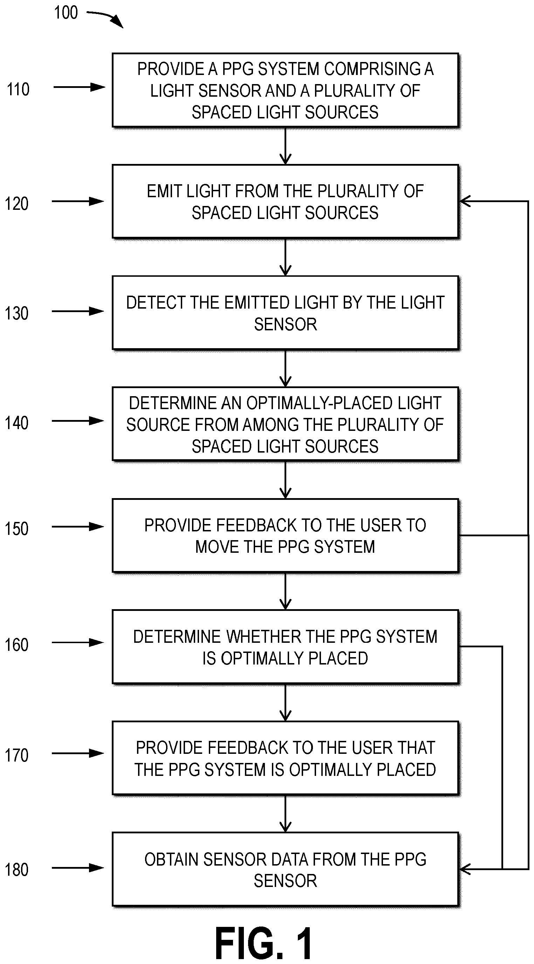

Turning to FIG. 1, a method 100 for providing optimal PPG sensor placement instructions to a user is provided according to one embodiment of the present disclosure. The method 100 includes: in a step 110, providing a PPG placement system comprising a plurality of light sources and at least one light sensor, wherein each of the plurality of light sources is spaced relative to the other plurality of light sources, and wherein at least one of the plurality of light sources and the at least one light sensor forms a PPG sensor; in a step 120, emitting light from each of the plurality of light sources, after the PPG placement system is placed on at least a first location on a surface of the subject's body; in a step 130, detecting by at least the one light sensor, the emitted light; in a step 140, determining which one of the plurality of light sources is most optimally placed on the surface of the subject's body using a processor of the PPG placement system; in a step 150, providing feedback to the user to move the PPG placement system in the direction of the one of the plurality of light sources that is determined to be most optimally placed on the surface of the subject's body via a user interface of the PPG placement system; in a step 160, determining whether the PPG is optimally placed on an optimal location on the surface of the subject's body; in a step 170, providing feedback to the user that the PPG placement system is optimally placed on an optimal location on the surface of the subject's body via the user interface of the PPG placement system; and in a step 180, obtaining sensor data from the subject via the PPG sensor of the PPG placement system. In particular embodiments, the user and the subject can be the same individual (i.e., the user of the PPG placement system is also the subject and is positioning the PPG sensor on a portion of their own body).

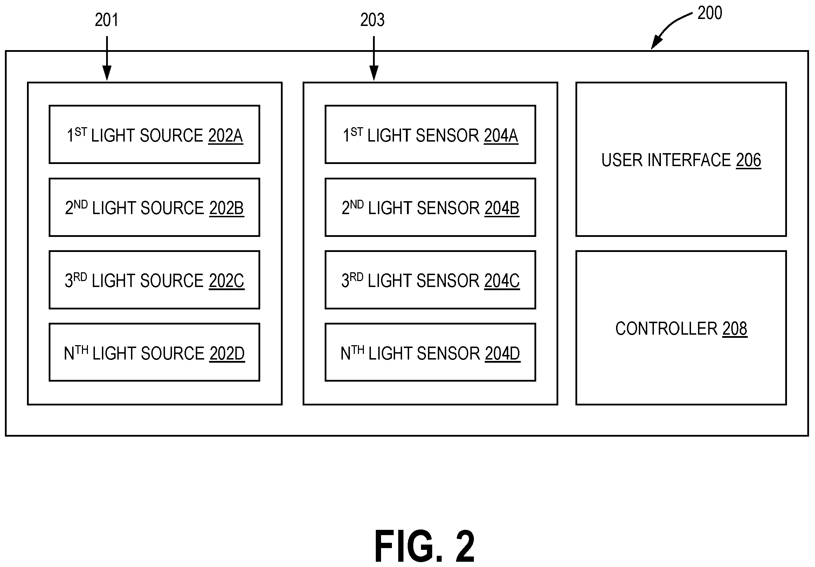

More specifically, in the step 110, the method 100 includes providing a PPG placement system comprising a plurality of light sources that are spaced relative to each other, and further comprises at least one light sensor. For example, with reference to FIG. 2, a block diagram illustrating a PPG placement system 200 according to certain aspects of the present disclosure is shown. In embodiments, the PPG placement system comprises an arrangement 201 of one or more light sources 202A, 202B, 202C, 202D, and an arrangement 203 of one or more light sensors 204A, 204B, 204C, 204D.

In some embodiments, the light sources 202A, 202B, 202C, 202D and light sensors 204A, 204B, 204C, 204D may be arranged 201, 203 in pairs, such that the number of light sensors 204A, 204B, 204C, 204D is equal to the number of light sources 202A, 202B, 202C, 202D. In such embodiments, the light emitted by each of the one or more light sources 202A, 202B, 202C, 202D may be exclusively sensed or otherwise measured by a corresponding light sensor 204A, 204B, 204C, 204D.

In other embodiments, the light sources 202A, 202B, 202C, 202D and the light sensors 204A, 204B, 204C, 204D may not be arranged 201, 203 in pairs. For example, according to certain embodiments, the light emitted by one of the light sources 202A, 202B, 202C, 202D may be sensed by a plurality of nearby light sensors 204A, 204B, 204C, 204D. According to other embodiments, the light emitted by a plurality of light sources 202A, 202B, 202C, 202D may be measured by a single light sensor 204A, 204B, 204C, 204D. In further embodiments, a combination of these arrangements 201, 203 may be utilized such that there are multiple light paths between a plurality of light sources 202A, 202B, 202C, 202D and a plurality of light sensors 204A, 204B, 204C, 204D.

In embodiments, one or more of the light sources 202A, 202B, 202C, 202D may be actuated consecutively in time (i.e., not simultaneously) such that light emitted from a light source 202A, 202B, 202C, 202D is sequentially measured by the one or more light sensors 204A, 204B, 204C, 204D.

In other embodiments, one or more of the light sources 202A, 202B, 202C, 202D may be actuated simultaneously such that two or more light sources 202A, 202B, 202C, 202D emit light at the same time and is simultaneously measured by the one or more light sensors 204A, 204B, 204C, 204D. For example, all of the light sources may emit light at the same time. According to an embodiment, when all of the light sources emit light at the same time, a broader light cone is made. Although all the light sources may emit light simultaneously, the received light intensity can be changing over the light sensors. Notably, light sources can be of different colors (including but not limited to green, red, and infrared, among others). Paths also change with color, with red deeper into the tissue and green more at the surface.

Accordingly, for example, the PPG placement system 200 may include one or more light sources 202A, 202B, 202C, 202D that are consecutively or simultaneously actuated to emit a desired light path, and one or more light sensors 204A, 204B, 204C, 204D that consecutively or simultaneously detect light emitted from at least one of the light sources 202A, 202B, 202C, 202D.

In embodiments, each of the one or more light sources 202A, 202B, 202C, 202D can be a light-emitting diode (LED) or another type of light-emitting element. In some embodiments, one or more of the light sources 202A, 202B, 202C, 202D can be a radiation source that emits incident radiation striking a subject. In embodiments, the incident radiation emitted by the light sources 202A, 202B, 202C, 202D can include, for example, light in the visible spectrum, infrared and/or near-infrared radiation, and/or ultraviolet radiation. In certain embodiments, the arrangement 201 of light sources 202A, 202B, 202C, 202D may include between about two and about nine light-emitting elements, although many other variations are possible.

In further embodiments, the arrangement 201 of light sources 202A, 202B, 202C, 202D comprises a single light-emitting element and optical elements (e.g., actuatable mirrors and/or prisms, etc.) to create the different light sources 202A, 202B, 202C, 202D having different positions and/or directions in which the light emanates. Put another way, the arrangement 201 may include one or more light-emitting elements that are shared between two or more light sources 202A, 202B, 202C, 202D via independent optical elements. In further embodiments, the one or more light sources 202A, 202B, 202C, 202D may be integrated together into an assembly such as a transmissive display.

In embodiments, each of the one or more light sensors 204A, 204B, 204C, 204D can be a photodiode or another type of light-sensitive element. Each light sensor 204A, 204B, 204C, 204D may be configured to sense, detect, or otherwise measure radiation emitted or reflected by a subject or subject. For example, incident radiation (including incident light) emitted from one or more of the light sources 202A, 202B, 202C, 202D towards a subject and absorbed and/or reflected by a portion of the subject's body. Based on the bodily tissues along the path of the emitted radiation (e.g., a light path, etc.), the radiation will be differently absorbed and/or scattered, thereby affecting the intensity of the reflected (transmitted) radiation, which can be measured by the one or more light sensors 204A, 204B, 204C, 204D.

As discussed above, the PPG placement system 200 can include pairs of light sensors 204A, 204B, 204C, 204D and light sources 202A, 202B, 202C, 202D such that the number of light sensors 204A, 204B, 204C, 204D is equal to the number of light sources 202A, 202B, 202C, 202D. That is, there is a light sensor 204A, 204B, 204C, 204D that corresponds to each of the light sources 202A, 202B, 202C, 202D. Accordingly, in some embodiments, the arrangement 203 of light sensors 204A, 204B, 204C, 204D can include between about one and about nine light-sensitive elements, although many other variations are possible.

In further embodiments, the arrangement 203 of one or more light sensors 204A, 204B, 204C, 204D may include a single light-sensitive element and optical elements (e.g., actuatable mirrors and/or prisms, etc.) to create different positions and/or directions from which emitted light may be captured. In such embodiments, each light sensor 204A, 204B, 204C, 204D may detect light emitted from two or more light sources 202A, 202B, 202C, 202D.

In particular embodiments, the light sensors 204A, 204B, 204C, 204D may be integrated together into an assembly such as an image sensor (e.g., a camera, etc.). In further embodiments, the light sources 202A, 202B, 202C, 202D and the light sensors 204A, 204B, 204C, 204D may be integrated together into a combined transmissive display and camera. For example, the arrangements 201, 203 may comprise thin foil technology, such as flexible photodiode arrays with integrated illumination.

In some embodiments, the arrangement 201, 203 of light sources 202A, 202B, 202C, 202D and light sensors 204A, 204B, 204C, 204D used to determine an optimal location for taking a PPG measurement (discussed in more detail below) may include the PPG sensor that is ultimately placed on the body of the subject. That is, at least one of the light sources 202A, 202B, 202C, 202D and at least one of the light sensors 204A, 204B, 204C, 204D may form a PPG sensor of the PPG placement system 200. However, in accordance with another embodiments, components other than the light sources 202A, 202B, 202C, 202D and the light sensors 204A, 204B, 204C, 204D may form a PPG sensor of the PPG placement system 200.

In other embodiments, the arrangement 201, 203 of light sources 202A, 202B, 202C, 202D and light sensors 204A, 204B, 204C, 204D used to determine an optimal location for taking a PPG measurement (discussed in more detail below) may be a stand-alone device that is separate from the PPG sensor ultimately placed on the body of the subject. That is, the arrangement 201, 203 of light sources 202A, 202B, 202C, 202D and light sensors 204A, 204B, 204C, 204D used to determine an optimal location for taking a PPG measurement may form a placement scanning device separate from the PPG sensor.

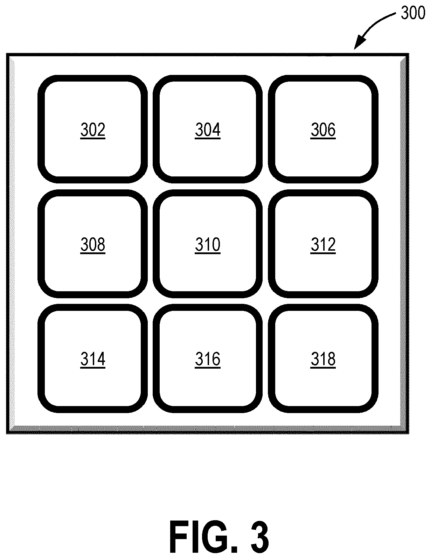

According to the present disclosure, the arrangement 201, 203 of light sources 202A, 202B, 202C, 202D and light sensors 204A, 204B, 204C, 204D is configured to generate a plurality of spatially diverse light paths such that a spatial gradient of the signal quality over the different light paths may be obtained. For example, with reference to FIG. 3, a PPG placement system 300 is illustrated according to one embodiment of the present disclosure. The PPG placement system 300 includes a plurality of light sources and light sensors arranged into a 3x3 matrix, thereby creating nine spatially diverse light paths at locations 302, 304, 306, 308, 310, 312, 314, 316, 318.

In embodiments, the arrangement 201, 203 of light sources and light sensors enables the generation of a spatial gradient of the signal quality over the spatially diverse light paths (e.g., locations 302, 304, 306, 308, 310, 312, 314, 316, 318). In some embodiments, the signal quality may be determined by distinguishing signal and noise components and determining a signal-to-noise ratio (SNR) for each of the spatially diverse light paths. A larger SNR indicates a better signal quality, and therefore a more optimal location to attach the PPG sensor. In embodiments, the signal and noise components may be separated by band-pass filtering the received signal, such that the pulse signal is passing (or, vice versa, is suppressed). The removed signal content represents the noise (or vice versa). Comparison of the two signal components can yield the SNR for signal quality. In further embodiments, the energy variance of the bandpass filtered signals may also or alternatively be computed, where the ratio represents the signal quality.

In some embodiments, the signal quality may be a metric based on the autocorrelation of the signals. More specifically, the signal quality may be determined based on the size of the autocorrelation at the first maximum, which occurs at the pulse interval between subsequent pulses. For example, the noisier the signal is, the lower this first maximum will be. As a result, the absolute and/or relative values can be observed, which may then be correlated to a fixed reference such as an ECG signal.

In some embodiments, the signal quality may be determined by measuring the size of the peak-to-valley in the pulse signals. Because this metric may be modulated by respiration of the subject, the values at the same phase in the respiration cycle would need to be compared in the matrix.

In some embodiments, the signal quality may be determined based on the AC over DC of the signals received using the arrangement 201, 203 of light sources 202A, 202B, 202C, 202D and light sensors 204A, 204B, 204C, 204D used to determine an optimal location for taking a PPG measurement.

In some embodiments, the signal quality may be determined based on the morphology of the signals receiving using the arrangement 201, 203 of light sources 202A, 202B, 202C, 202D and light sensors 204A, 204B, 204C, 204D used to determine an optimal location for taking a PPG measurement. For example, at a more optimal placement, a clean waveform is more likely to appear with clearly defined peaks and valleys.

Another metric could be the morphology of the PPG signal. At proper placement, a clean wave form appears with peaks and valleys.

In some embodiments, the spatial gradient of the signal quality over the spatially diverse light paths may be generated that on a scale of 0 to 1, with 0 representing the lowest quality signal (i.e., no signal), and 1 representing the best signal. In particular embodiments, the spatial gradient of the signal quality may be represented digitally in a various of data structures, including but not limited to a twodimensional array or matrix. For example, the following matrix may be generated and stored in accordance with the PPG placement system 300 shown in FIG. 3:

$$\begin{bmatrix} s_1 & s_2 & s_3 \\ s_4 & s_5 & s_6 \\ s_7 & s_8 & s_9 \end{bmatrix}$$

where $s_i$ is a decimal between 0 and 1, I is an integer corresponding to a unique spatially diverse light path, and each entry ($s_i$) corresponds to one of the spatially diverse light paths at locations 302, 304, 306, 308, 310, 312, 314, 316, 318.

Although the arrangement 201, 203 of the light sources and light sensors illustrated in the PPG placement system of FIG. 3 includes a square distribution of components, it is contemplated that other arrangements 201, 203, including other shapes and other numbers of elements, may be used. For example, and without limitation, a cross-shaped arrangement may be utilized and a corresponding spatial gradient formed, or a circular arrangement may be utilized and a corresponding spatial gradient formed.

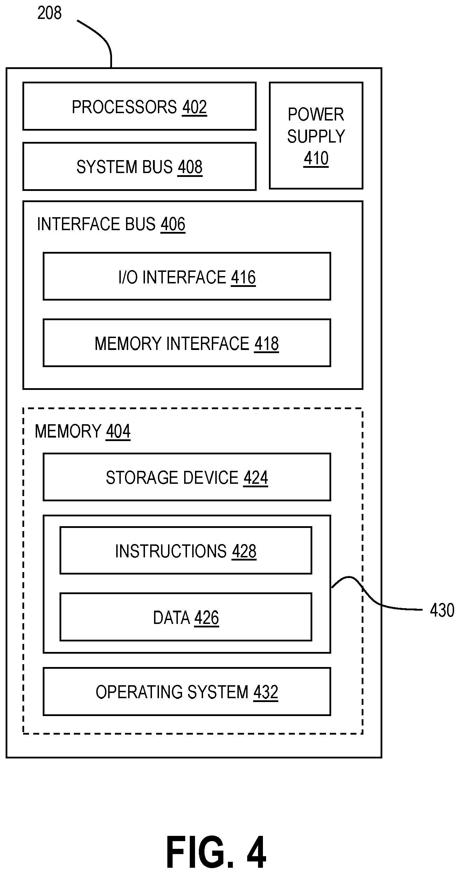

In embodiments, the PPG placement system 200, 300 further comprises a controller 208 having one or more processors (e.g., processors 402 shown in FIG. 4) configured to operate the light sources 202A, 202B, 202C, 202D and/or light sensors 204A, 204B, 204C, 204D, and further configured to perform one or more other steps of the methods described herein.

In certain embodiments, the one or more processors of the controller 208 may be configured to at least determine a direction in which the PPG placement system 200, 300 may be moved relative to the surface of the subject's body in order to obtain an improved (i.e. higher quality) signal. In some embodiments, the direction may be determined based on the spatial gradient of the signal quality over the spatially diverse light paths. For example, the following spatial gradient representation could indicate that the PPG placement system 200, 300 should be moved towards the location corresponding to the "0.6" reading and away from location corresponding to the "0.1" reading:

$$\begin{bmatrix} 0.6 & 0.5 & 0.3 \\ 0.5 & 0.3 & 0.2 \\ 0.3 & 0.2 & 0.1 \end{bmatrix}$$

In some embodiments, the one or more processors of the controller 208 may be configured to at least determine a distance that the PPG placement system 200, 300 may be moved relative to the surface of the subject's body in order to obtain an improved (i.e. higher quality) signal. In some embodiments, the distance may be determined based on the spatial gradient of the signal quality over the spatially diverse light paths. For example, the distance that the PPG placement system 200, 300 should be moved relative to the surface of the subject's body may be based on the statistical features of the spatial gradient of the signal quality over the spatially diverse light paths, including but not limited to, the spatial relationship between the min/max values of the spatial gradient.

In embodiments, the PPG placement system 200 further comprises a user interface 206 configured to provide feedback to a subject, and the one or more processors of the controller 208 can be further configured to operate the user interface 206 accordingly. In particular embodiments, the user interface 206 of the PPG placement system 200 may be configured to provide feedback to the subject indicating the direction and/or distance that the PPG placement system 200 should be moved in order to obtain an improved signal. For example, the feedback provided may be at least one of audible feedback, haptic feedback, visual feedback, and text-based feedback.

According to an embodiment, the user interface 206 can provide feedback to the user indicating that the optimal location has been achieved or located. That feedback can be audible feedback, haptic feedback, visual feedback, and text-based feedback, among other types of feedback. According to one embodiment, the feedback can be the cessation of a feedback signal. For example, the device can emit a signal such as a light or a noise or a vibration, and can stop emitting that signal once optimal placement is achieved.

With reference to FIG. 4, a block diagram of a PPG controller 208 that may form a part of a PPG placement system 200, 300 is illustrated according to aspects of the present disclosure. In particular, each PPG placement system 200, 300 may include a PPG controller 208 that is operatively connected to one or more components of the PPG placement system 200, 300, including but not limited to, the one or more light sources 202A, 202B, 202C, 202D, the one or more light sensors 204A, 204B, 204C, 204D, and/or the user interface 206. The PPG controller 208 may be configured to operate PPG placement system 200, 300 and/or one or more components thereof.

In the example of FIG. 4, the PPG controller 208 can include one or more processors 402, machine-readable memory 404, and an interface bus 406, all of which may be interconnected and/or communicate through a system bus 408 containing conductive circuit pathways through which instructions (e.g., machine-readable signals) may travel to effectuate communication, tasks, storage, and the like. The PPG controller 208 may be connected to a power source 410, which can include an internal power supply and/or an external power supply.

The one or more processors 402 may include a high-speed data processor adequate to execute the program components described herein and/or various specialized processing units as may be known in the art. In some examples, the one or more processors 402 may be a single processor, multiple processors, or multiple processor cores on a single die.

In some examples, the interface bus 406 may include an input/output ("I/O") interface 416 configured to connect and communicate with one or more connected electronic devices, and/or a memory interface 418 configured to accept, communication, and/or connect to a number of machine-readable memory devices (e.g., memory 404).

The memory 404 can be variously embodied in one or more forms of machine-accessible and machine-readable memory. In some examples, the memory 404 includes a storage device 424 comprises one or more types of memory. For example, the storage device 424 can include, but is not limited to, a non-transitory storage medium, a magnetic disk storage, an optical disk storage, an array of storage devices, a solid-state memory device, and the like, including combinations thereof.

Generally, the memory 404 is configured to store data / information 426 and instructions 428 that, when executed by the one or more processors 402, causes the PPG controller 208 to perform one or more tasks. In particular examples, the memory 404 includes a PPG controller package 430 that comprises a collection of program components, database components, and/or data configured to, when executed by the one or more processors 402, cause the PPG controller 208 to perform one or more of the steps of method 100. According to the present disclosure, the PPG controller package 430 may include, but is not limited to, instructions 428 having one or more software packages configured to perform one or more of the steps of method 100. These software packages may be incorporated into, loaded from, loaded onto, or otherwise operatively available to and from the PPG controller 208. Put another way, the PPG controller package 430 and/or one or more software packages may be incorporated into, loaded from, loaded onto, or otherwise operatively available to a PPG placement system 200, 300. In embodiments, the PPG controller 208 includes at least an operating system component 432, which may be stored in the memory 404. The operating system component 432 may be an executable program facilitating the operation of the PPG controller package 430. Typically, the operating system component 432 can facilitate access of the I/O interface and memory interface, and can communicate with other components of the PPG placement system 200, 300.

Returning to FIG. 1, after providing a PPG placement system 200, 300 as described herein, the method 100 may include a step of determining that the PPG placement system 200, 300 has been placed on a surface of the subject's body. Then, the method 100 can include performing one or more iterations of: in a step 120, emitting light from each of the plurality of light sources 202A, 202B, 202C, 202D, after the PPG placement system 200, 300 is placed on at least a first location on a surface of the subject's body; in a step 130, detecting by at least the one light sensor 204A, 204B, 204C, 204D, the emitted light; in a step 140, determining which one of the plurality of light sources 202A, 202B, 202C, 202D is most optimally placed on the surface of the subject's body using a processor 402 of the PPG placement system 200, 300; and in a step 150, providing feedback to a user to move the PPG placement system 200, 300 in the direction of the one of the plurality of light sources 202A, 202B, 202C, 202D that is determined to be most optimally placed on the surface of the subject's body via a user interface 206 of the PPG placement system 200, 300.

More specifically, in the step 120, light and/or another form of radiation may be emitted from one or more of the light sources 202A, 202B, 202C, 202D, as described above. In particular embodiments, the PPG controller package 430 includes instructions 428 having a radiation emitting component, which may be a stored program component that, when executed by at least one processor (e.g., the one or more processors 402 of the PPG controller 208), directs the one or more light sources 202A, 202B, 202C, 202D to emit light and/or other forms of radiation in a predetermined pattern after the PPG placement system 200, 300 is placed on at least a first location on a surface of the subject's body.

After emitting light and/or another form of radiation in accordance with the present disclosure, the method 100 includes, in the step 130, detecting by at least the one light sensor 204A, 204B, 204C, 204D, the emitted light and/or other radiation. In embodiments, detecting the emitted light and/or other radiation using the at least one light sensor 204A, 204B, 204C, 204D includes detecting light and/or other radiation that was emitted by the one or more light sources 202A, 202B, 202C, 202D and reflected by the tissues of the subject. In particular embodiments, the PPG controller package 430 includes instructions 428 having a radiation detection component, which may be a stored program component that, when executed by at least one processor (e.g., the one or more processors 402 of the PPG controller 208), instructs the one or more light sensors 204A, 204B, 204C, 204D to received, measure, or otherwise detect light and/or other forms of radiation in the form of sensor data.

In the step 140, the method 100 includes determining, by a processor 402 of the PPG placement system 200, 300, which one of the plurality of light sources 202A, 202B, 202C, 202D is most optimally placed on the surface of the subject's body. In embodiments, determining which of the plurality of light sources is most optimally placed on the surface of the subject's body includes a comparison of signal quality of the light emitted by each of the plurality of light sources 202A, 202B, 202C, 202D and received by the one or more light sensors 204A, 204B, 204C, and 204D. In particular embodiments, the PPG controller package 430 includes instructions 428 having an optimal position component, which may be a stored program component that, when executed by at least one processor (e.g., the one or more processors 402 of the PPG controller 208), analyzes the signal quality of the light emitted by each of the plurality of light sources 202A, 202B, 202C, 202D and received by the one or more light sensors 204A, 204B, 204C, and 204D. For example, in embodiments, the step 140 can include generating a spatial gradient of the signal quality over a spatially diverse light paths (e.g., locations 302, 304, 306, 308, 310, 312, 314, 316, 318 shown in FIG. 3).

In the step 150, the method 100 includes providing feedback to a user, via a user interface 206 of the PPG placement system 200, 300, indicating that the PPG placement system 200, 300 should be moved in the direction of the most optimally placed light source 202A, 202B, 202C, 202D determined in step 140. In particular embodiments, the PPG controller package 430 can include instructions 428 having a feedback component, which may be a stored program component that, when executed by at least one processor (e.g., the one or more processors 402 of the PPG controller 208), operates the user interface 206 to provide feedback to the user as described herein.

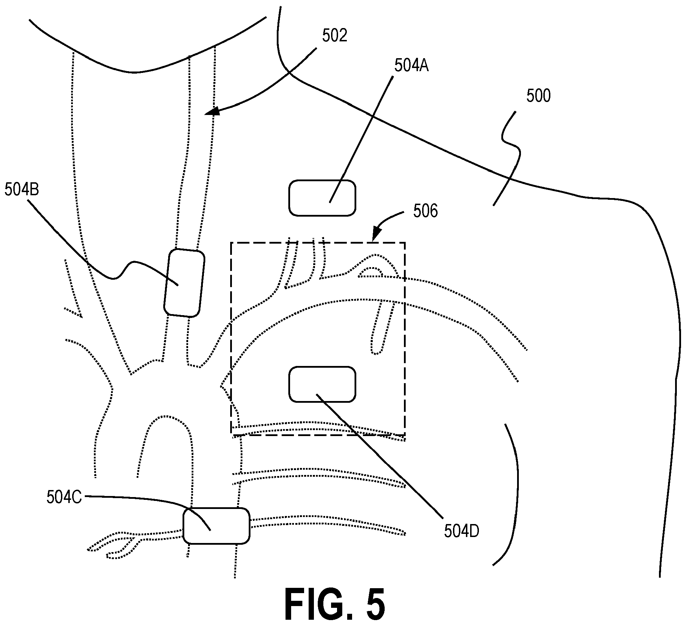

In embodiments, step 120 through step 150 may be performed iteratively a plurality of times in order to guide a user to properly place the PPG placement system 200, 300 relative to an optimal location on a subject. For example, with reference to FIG. 5, the upper body of a subject 500 is illustrated with parts of the circulatory system 502 of the subject 500 shown. In the example of FIG. 5, multiple PPG placement systems 504A, 504B, 504C, 504D are placed on a surface of the body of the subject 500 in different locations. As shown, certain PPG placement systems 504B, 504C are placed directly over and/or adjacent to a major artery of the circulatory system 502 of the subject 500, which generally provides a high-quality signal. In contrast, certain PPG placement systems 504A, 504D are not placed over and/or adjacent to a major artery, but may be nearby a smaller arteriole instead, which generally results in a reduced signal quality. According to the present disclosure, optimal PPG sensor placement instructions may be provided to a user in order to avoid improperly positioned sensors like PPG placement systems 504A, 504D.

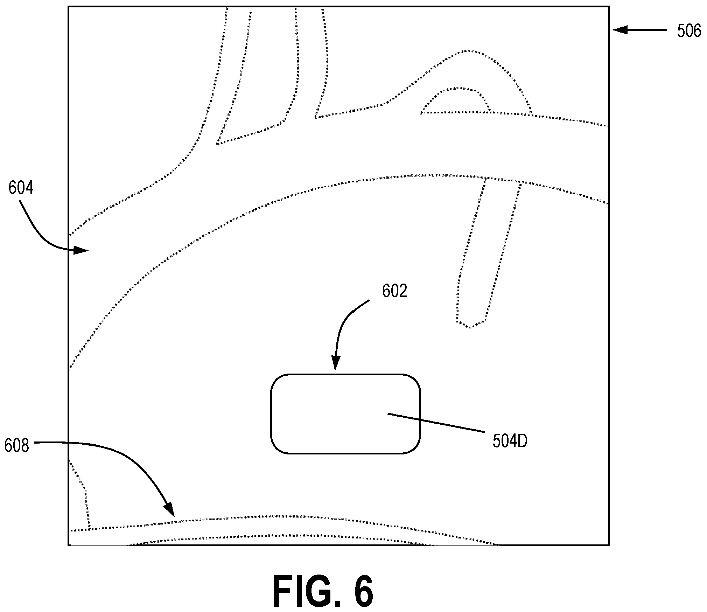

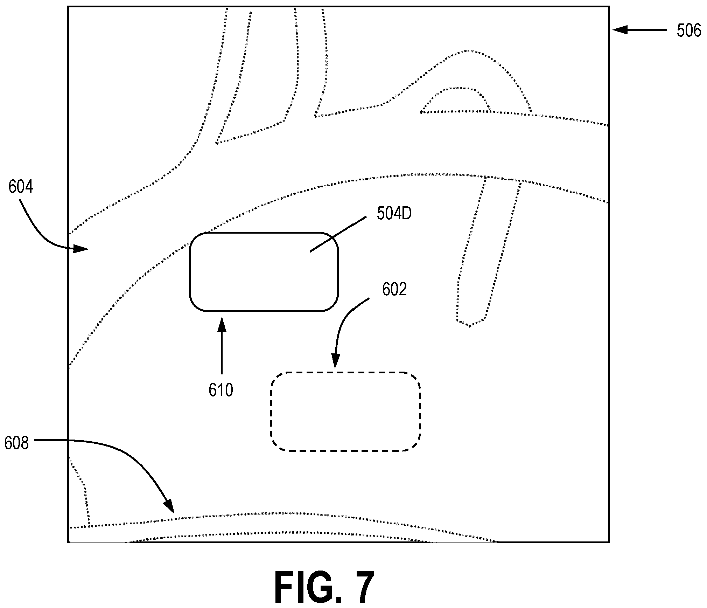

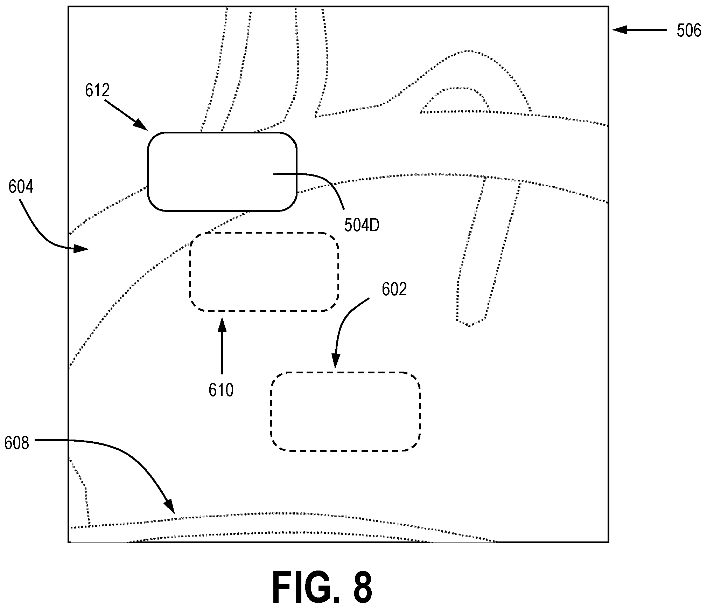

For example, with reference to FIGS. 6-8, the enlarged view of FIG. 5 is shown with respect to the region 506. As shown in FIG. 6, the poorly placed PPG placement system 504D is located at a first position 602 nearby a small arteriole 608. According to the present disclosure, step 120 through step 150 of the method 100 may be performed to determine at least a second position 610 where improved signal quality can be obtained (e.g., closer to a larger artery 604), as illustrated in FIG. 7. As described above, step 120 through step 150 of the method 100 may be iteratively performed such that at least a third position 612 where still further improved signal quality can be obtained, as illustrated in FIG. 8.

After step 120 through step 150 is performed one or more times, the method 100 can include, in a step 160, determining whether the PPG placement system 200, 300 is optimally placed on an optimal location on the surface of the subject's body. In particular embodiments, the optimal position component of the PPG controller package 430, when executed by at least one processor (e.g., the one or more processors 402 of the PPG controller 208), can further determine whether the PPG sensor is optimally placed on an optimal location on the surface of the subject's body. In embodiments, this determination may be made based on a predetermined and/or customizable threshold indicating a sufficient signal quality given an intended purpose or use case. Put another way, the step 160 can include determining when a sufficiently optimal location for the PPG sensor is found.

In the step 170, the method 100 includes providing feedback to a user via the user interface 206 of the PPG placement system 200, 300 that the PPG sensor is optimally placed on an optimal location on the surface of the subject's body. In particular embodiments, the feedback component of the PPG controller package 430 can include instructions 428 that, when executed by at least one processor (e.g., the one or more processors 402 of the PPG controller 208), operates the user interface 206 to provide the desired feedback to the user as described herein.

In the step 180, the method 100 includes obtaining sensor data from the subject via the PPG sensor. As discussed above, the PPG sensor may form part of the PPG placement system 200, 300 or may be a separate, stand-alone device.

In accordance with an embodiment, step 180 of the method can occur after, for example, any of steps 150, 160, or 170 of the method. For example, the PPG sensor can obtain sensor data from the subject during and/or after step 150 when the system provides feedback to the user that the PPG placement system should be moved in the direction of the most optimally placed light source 202A, 202B, 202C, 202D determined in step 140. Obtaining sensor data during and/or after step 150 can be automatic, or can be the result of a user action. As another example, the PPG sensor can obtain sensor data from the subject during and/or after step 160 when the system determines that the PPG placement system is optimally placed on an optimal location on the surface of the subject's body. Obtaining sensor data during and/or after step 160 can be automatic, or can be the result of a user action. As another example, the PPG sensor can obtain sensor data from the subject during and/or after step 170, when the system provides feedback to the user that the PPG placement system is optimally placed on an optimal location on the surface of the subject's body. Obtaining sensor data during and/or after step 170 can be automatic, or can be the result of a user action.

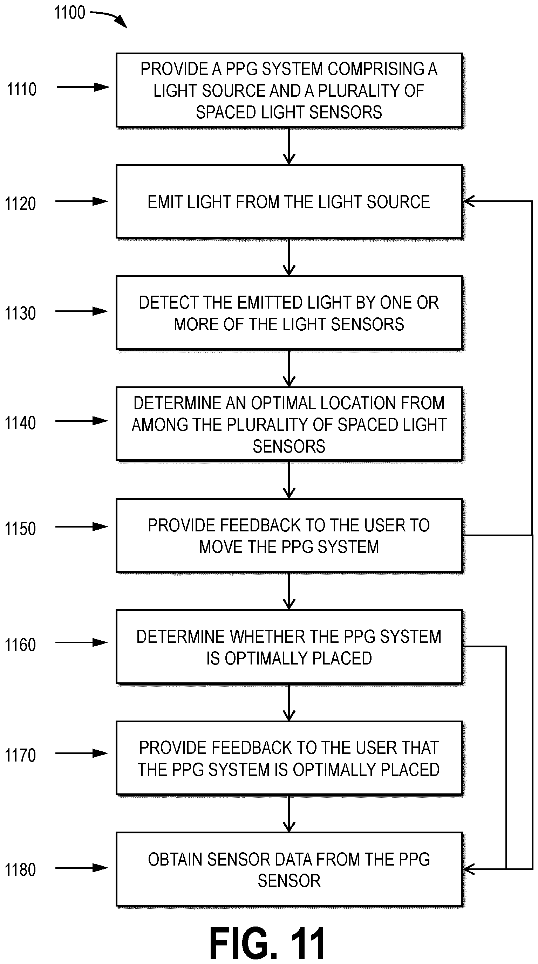

Referring to an alternative embodiment of the method described in conjunction with FIG. 1 is the method 1100 illustrated in FIG. 11. In step 1110, a PPG placement system comprising at least one light source (202A, 202B, 202C, 202D) is provided, the PPG placement system further comprising a plurality of light sensors (204A, 204B, 204C, 204D).

At step 1120 of the method, after providing a PPG placement system as described herein, the method 1100 may optionally include a step of determining that the PPG placement system has been placed on a surface of the subject's body. Then, the method 1100 can include performing one or more iterations of: in a step 1120, emitting light from the at least one light source 202A, 202B, 202C, 202D, after the PPG placement system is placed on at least a first location on a surface of the subject's body.

At step 1130 of the method, the plurality of light sensors 204A, 204B, 204C, 204D, detect the emitted light. According to an embodiment, the plurality of light sensors enable detection of the best or optimal light path for PPG sensor placement/detection, even when light is emitted by only a single light source (such as 202A, etc.). In step 1140, the system determines, from the light path(s) detected by the plurality of sensors, optimum placement of a PPG sensor on the surface of the subject's body. In step 1150, the PPG placement system provides feedback to the user to move the system in the direction of the optimal light path.

In optional step 1160 of the method, the system determines that the PPG is optimally placed on an optimal location on the surface of the subject's body, and in optional step 1170 of the method the system provides feedback to the user, via the user interface of the PPG placement system, that the PPG placement system is optimally placed on the optimal location on the surface of the subject's body. In optional step 1180 of the method, a PPG sensor obtains sensor data from the subject.

Also provided herein are methods for providing optimal PPG sensor placement instructions to a user, wherein the method involves emitting light from one or more of at least one light sources of a PPG placement system at a plurality of different locations on a surface of the subject's body, and then detecting the emitted light using at least one light sensor of the PPG placement system at each of the plurality of different locations. More specifically, in embodiments, the user may perform an emission-detection operation over a target region of the subject's body using a PPG placement system while tracking the location of the PPG placement system in order to generate a scan matrix of the target region. Within the target region scanned by the user, an optimal location for placing a PPG sensor may be determined.

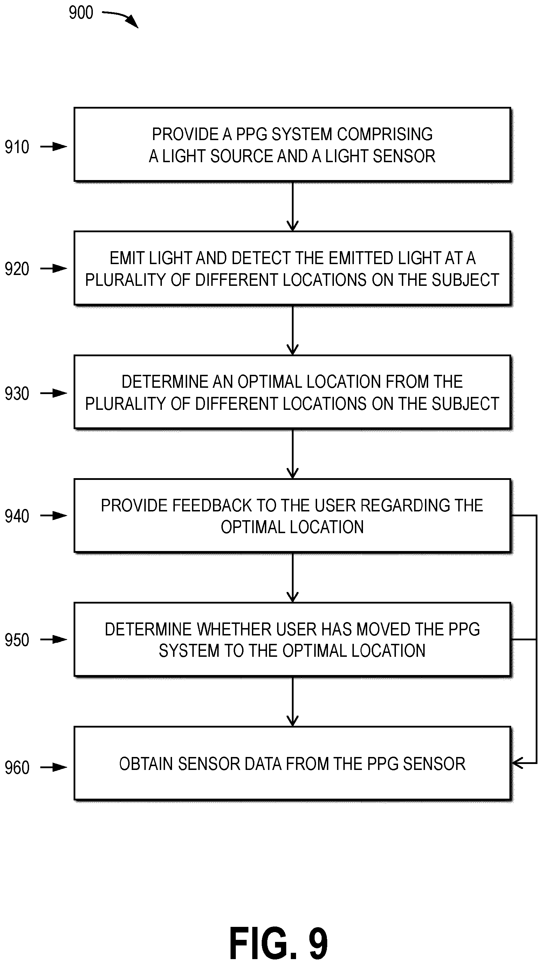

For example, with reference to FIG. 9, a method 900 for providing optimal PPG sensor placement instructions to a user is illustrated. In a step 910, the method 900 comprises providing a PPG placement system 200, 300 comprising at least one light source 202A, 202B, 202C, 202D and at least one light sensor 204A, 204B, 204C, 204D. According to an embodiment, one or more of the at least one light source 202A, 202B, 202C, 202D and the at least one light sensor 204A, 204B, 204C, 204D forms a PPG sensor, although other components may form the PPG sensor.

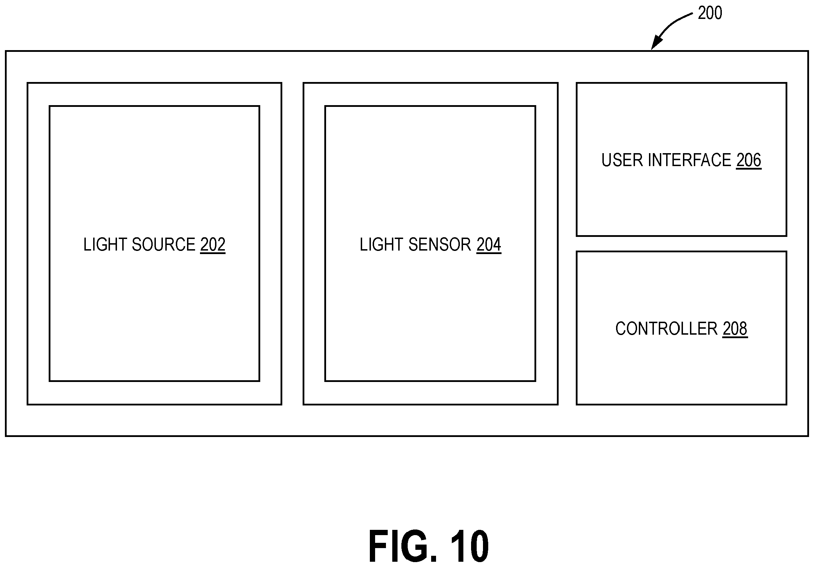

For example, with reference to FIG. 10, is an embodiment of a PPG placement system 200 according to certain aspects of the present disclosure is shown. In embodiments, the PPG placement system comprise a light source 202 and a light sensor 204. In other embodiments, the PPG placement system can comprise a plurality of light sources 202A, 202B, 202C, 202D and one or more light sensors 204A, 204B, 204C, 204D.

The method 900 comprises, in a step 920, performing, at each of a plurality of different locations on a surface of the subject's body the steps of: (i) emitting light from the one or more light sources; and (ii) detecting, by the at least one light sensor 204, the emitted light.

In embodiments, the step 920 may include detecting, determining, and/or otherwise recording the position of each of the plurality of different locations on the surface of the subject's body where the emission-detection operation takes place. For example, the PPG placement system 200, 300 may further include a camera facing the targeted region of the subject's body and an image processing means enabled to assign spatial coordinates to different locations of the PPG placement system 200, 300 on the body of the subject while scanning (i.e., performing the emission-detection operations).

In other embodiments, the PPG placement system 200, 300 may further include a projector and a camera (e.g., an infrared camera) configured such that the project projects a grid onto the target surface of the subject's body and, using an image processing means, assigns spatial coordinates to different locations of within that area.

In further embodiments, the PPG placement system 200, 300 may include one or more beacons positioned on or around the subject's body, and may utilize a time-difference of arrival (TDOA) or similar technique to assign spatial coordinates to the different emission-detection scans performed. For example, in some embodiments, a radio-frequency (RF)-based localization device may utilize a greater than 1 GHz bandwidth to enable millimeter-scale spatial accuracy. In specific embodiments, ultra-wide band (UWB) and/or 60 GHZ technology may be used.

In embodiments, the step 920 may also include generating a signal quality matrix over the entire area covered by the plurality of different locations on the surface of the subject's body.

In a step 930, the method 900 comprises determining which one of plurality of different locations on the surface of the subject's body is a most optimal placement of the PPG sensor based on the signal quality quantified in the step 920. In embodiments, step 930 can include determining which one of the plurality of different locations on the surface of the subject's body is a most optimal placement via a processor 402 of the PPG placement system 200, 300. As described above, one or more metrics may be utilized to evaluate the signal quality.

In a step 940, the method 900 comprises providing feedback to the user, via a user interface 206 of the PPG placement system 200, 300, regarding the most optimal placement of the PPG placement system.

In a step 950, the method 900 comprises determining that the user has moved the PPG placement system 200, 300 to the most optimal placement.

In a step 960, the method 900 comprises obtaining sensor data from the subject via the PPG sensor of the PPG placement system 200, 300.

In accordance with an embodiment, step 960 of the method can occur after, for example, any of steps 940 or 950 of the method. For example, the PPG sensor can obtain sensor data from the subject during and/or after step 940 when the system provides feedback to the user regarding the most optimal placement of the PPG placement system. Obtaining sensor data during and/or after step 940 can be automatic, or can be the result of a user action. As another example, the PPG sensor can obtain sensor data from the subject during and/or after step 950 of the method, when the system determines that the user has moved the PPG placement system to the most optimal placement. Obtaining sensor data during and/or after step 950 can be automatic, or can be the result of a user action.

It should be appreciated that all combinations of the foregoing concepts and additional concepts discussed in greater detail herein (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, fieldprogrammable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

**Claims**

1. A method (100) for providing optimal photoplethysmography (PPG) sensor placement instructions to a user, comprising:

    providing (110) a PPG placement system (200, 300) comprising a plurality of light sources (202A, 202B, 202C, 202D) and at least one light sensor (204A, 204B, 204C, 204D), wherein each of the plurality of light sources is spaced relative to the other plurality of light sources; performing one or more iterations of:

    emitting (120) light from each of the plurality of light sources, after the PPG placement system is placed on a first location on a surface of the subject's body;
    detecting (130), by the at least one light sensor, the emitted light;
    determining (140), by a processor (208) of the PPG placement system, which one of the plurality of light sources is most optimally placed on the surface of the subject's body; and
    providing (150) feedback to the user, via a user interface (206) of the PPG placement system, to move the PPG placement system in the direction of the one of the plurality of light sources that is determined to be most optimally placed on the surface of the subject's body.

2. The method of claim 1, further comprising the steps of:

    determining (160) that the PPG sensor is optimally placed on an optimal location on the surface of the subject's body; and
    providing (170) feedback to the user, via the user interface (206) of the PPG placement system, that the PPG sensor is optimally placed on an optimal location on the surface of the subject's body.

3. The method of claim 1, further comprising the step of obtaining (180) sensor data from the subject via a PPG sensor of the PPG placement system.

4. The method of claim 1, wherein determining which of the plurality of light sources is most optimally placed on the surface of the subject's body comprises a comparison of signal quality of the emitted light received from each of the plurality of light sources.

5. The method of claim 1, wherein the feedback is one or more of audible feedback, haptic feedback, visual feedback, and text-based feedback.

6. The method of claim 1, wherein light is emitted sequentially from each of the plurality of light sources during the emitting step.

7. The method of claim 1, wherein light is emitted from the plurality of light sources by an arrangement of a single light source.

8.  A method (900) for providing optimal photoplethysmography (PPG) sensor placement instructions to a user, comprising:

    providing (910) a PPG placement system (200, 300) comprising at least one light source (202A, 202B, 202C, 202D) and at least one light sensor (204A, 204B, 204C, 204D);
    performing (920), at each of a plurality of different locations on a surface of the subject's body the steps of: (i) emitting light from the at least one light source; and (ii) detecting, by the at least one light sensor, the emitted light;
    determining (930), by a processor (402) of the PPG placement system, which one of plurality of different locations on the surface of the subject's body is a most optimal placement of the PPG placement system for signal quality; and
    providing (940) feedback to the user, via a user interface (206) of the PPG placement system, regarding the most optimal placement of the PPG placement system.

9.  The method of claim 8, further comprising the step of determining (950) that the user has moved the PPG placement system to the most optimal placement.

10. The method of claim 8, further comprising the step of obtaining (960) sensor data from the subject via a PPG sensor, after determining that the user has moved the PPG placement system to the most optimal placement.

11. A method (1100) for providing optimal photoplethysmography (PPG) sensor placement instructions to a user, comprising:

    providing (1110) a PPG placement system (200, 300) comprising at least one light source (202A, 202B, 202C, 202D) and a plurality of light sensors (204A, 204B, 204C, 204D), wherein each of the plurality of light sensors is spaced relative to the other plurality of light sensors;
    performing one or more iterations of:

        emitting (1120) light from at least one light source, after the PPG placement system is placed on a first location on a surface of the subject's body;
        detecting (1130), by one or more of the plurality of light sensors, the emitted light;
        determining (1140), by a processor (208) of the PPG placement system based on the detected emitted light, an optimal placement for a PPG sensor on the surface of the subject's body; and
        providing (1150) feedback to the user, via a user interface (206) of the PPG placement system, to move the PPG placement system in the direction of the determined optimal placement on the surface of the subject's body.

12. The method of claim 11, further comprising the steps of:

    determining (1160) that the PPG sensor is optimally placed on an optimal location on the surface of the subject's body; and
    providing (1170) feedback to the user, via the user interface (206) of the PPG placement system, that the PPG sensor is optimally placed on an optimal location on the surface of the subject's body.

13. The method of claim 11, further comprising the step of obtaining (1180) sensor data from the subject via a PPG sensor of the PPG placement system.

14. The method of claim 11, wherein determining an optimal placement for a PPG sensor on the surface of the subject's body comprises a comparison of the emitted light received by each of the plurality of light sensors.

15. The method of claim 11, wherein the feedback is one or more of audible feedback, haptic feedback, visual feedback, and text-based feedback.

FIG. 1

EP 4 378 387 A1

201                                     203                                          200

1ST LIGHT SOURCE 202A          1ST LIGHT SENSOR 204A

2ND LIGHT SOURCE 202B          2ND LIGHT SENSOR 204B          USER INTERFACE 206

3RD LIGHT SOURCE 202C          3RD LIGHT SENSOR 204C

NTH LIGHT SOURCE 202D          NTH LIGHT SENSOR 204D          CONTROLLER 208

# FIG. 2

300

| | | |
|---|---|---|
| 302 | 304 | 306 |
| 308 | 310 | 312 |
| 314 | 316 | 318 |

# FIG. 3

208

PROCESSORS 402

POWER SUPPLY 410

SYSTEM BUS 408

INTERFACE BUS 406

I/O INTERFACE 416

MEMORY INTERFACE 418

MEMORY 404

STORAGE DEVICE 424

INSTRUCTIONS 428

DATA 426

430

OPERATING SYSTEM 432

# FIG. 4

FIG. 5

EP 4 378 387 A1

EP 4 378 387 A1

FIG. 6

506

504D

602

610

604

608

**FIG. 7**

**FIG. 8**

EP 4 378 387 A1

900

910 → PROVIDE A PPG SYSTEM COMPRISING
A LIGHT SOURCE AND A LIGHT SENSOR

920 → EMIT LIGHT AND DETECT THE EMITTED LIGHT AT A
PLURALITY OF DIFFERENT LOCATIONS ON THE SUBJECT

930 → DETERMINE AN OPTIMAL LOCATION FROM THE
PLURALITY OF DIFFERENT LOCATIONS ON THE SUBJECT

940 → PROVIDE FEEDBACK TO THE USER REGARDING THE
OPTIMAL LOCATION

950 → DETERMINE WHETHER USER HAS MOVED THE PPG
SYSTEM TO THE OPTIMAL LOCATION

960 → OBTAIN SENSOR DATA FROM THE PPG SENSOR

# FIG. 9

**FIG. 10**

EP 4 378 387 A1

1100

1110 → PROVIDE A PPG SYSTEM COMPRISING A LIGHT SOURCE AND A PLURALITY OF SPACED LIGHT SENSORS

1120 → EMIT LIGHT FROM THE LIGHT SOURCE

1130 → DETECT THE EMITTED LIGHT BY ONE OR MORE OF THE LIGHT SENSORS

1140 → DETERMINE AN OPTIMAL LOCATION FROM AMONG THE PLURALITY OF SPACED LIGHT SENSORS

1150 → PROVIDE FEEDBACK TO THE USER TO MOVE THE PPG SYSTEM

1160 → DETERMINE WHETHER THE PPG SYSTEM IS OPTIMALLY PLACED

1170 → PROVIDE FEEDBACK TO THE USER THAT THE PPG SYSTEM IS OPTIMALLY PLACED

1180 → OBTAIN SENSOR DATA FROM THE PPG SENSOR

# FIG. 11

Europäisches
Patentamt

European
Patent Office

Office européen
des brevets

# EUROPEAN SEARCH REPORT

Application Number

**EP 22 21 0706**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/060807 A1 (OISHI YOSHIHIRO [JP]) 15 March 2007 (2007-03-15) * paragraphs [0060], [0089], [0123] – [0126]; figure 5B * | 1-15 | INV. A61B5/1455 |
| X | EP 3 430 980 A1 (KONINKLIJKE PHILIPS NV [NL]) 23 January 2019 (2019-01-23) | 8-10 | |
| A | * paragraphs [0001], [0069] – [0080] * | 1-7, 11-15 | |
| A | US 2019/008432 A1 (BASHAN OHAD [IL] ET AL) 10 January 2019 (2019-01-10) * paragraphs [0094], [0095] * | 1,6 | |

TECHNICAL FIELDS
SEARCHED (IPC)

**A61B**

The present search report has been drawn up for all claims

| Place of search | Date of completion of the search | Examiner |
|---|---|---|
| **The Hague** | **30 March 2023** | **Knüpling, Moritz** |

EPO FORM 1503 03.82 (P04C01)

## ANNEX TO THE EUROPEAN SEARCH REPORT
## ON EUROPEAN PATENT APPLICATION NO.

EP 22 21 0706

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2023

| Patent document cited in search report | | | Publication date | Patent family member(s) | | | Publication date |
|---|---|---|---|---|---|---|---|
| US 2007060807 | A1 | | 15-03-2007 | CN | 1919137 | A | 28-02-2007 |
| | | | | JP | 4607709 | B2 | 05-01-2011 |
| | | | | JP | 2007054497 | A | 08-03-2007 |
| | | | | KR | 20070024432 | A | 02-03-2007 |
| | | | | US | 2007060807 | A1 | 15-03-2007 |
| | | | | | | | |
| EP 3430980 | A1 | | 23-01-2019 | EP | 3430980 | A1 | 23-01-2019 |
| | | | | WO | 2019016192 | A1 | 24-01-2019 |
| | | | | | | | |
| US 2019008432 | A1 | | 10-01-2019 | AU | 2016381563 | A1 | 19-07-2018 |
| | | | | CA | 3010164 | A1 | 06-07-2017 |
| | | | | CN | 108601529 | A | 28-09-2018 |
| | | | | EP | 3397139 | A1 | 07-11-2018 |
| | | | | IL | 260295 | A | 31-07-2018 |
| | | | | JP | 2019506205 | A | 07-03-2019 |
| | | | | US | 2019008432 | A1 | 10-01-2019 |
| | | | | WO | 2017115361 | A1 | 06-07-2017 |

EPO FORM P0459

For more details about this annex : see Official Journal of the European Patent Office, No. 12/82